# EUROPEAN PATENT SPECIFICATION

(11) **EP 2 332 469 B1**
(45) Date of publication and mention of the grant of the patent: **27.05.2015**
(21) Application number: 09809895.7
(22) Date of filing: 25.08.2009
(51) Int. Cl.: A61B 17/02, A61B 17/00, A61L 31/00

(54) **ENDOSCOPE EXCLUDER**
ENDOSKOP MIT EXCLUDER
BANDE ENDOSCOPIQUE POUR REPOUSSER UN ORGANE

(30) Priority: 01.09.2008 JP 2008223986
(43) Date of publication of application: 15.06.2011
(73) Proprietor: Kawamoto Corporation, Osaka 540-0022 (JP)
(72) Inventor: TSUCHIDA, Shinobu, Kobe-shi Hyogo 658-0032 (JP); HAMAGUCHI, Takeyuki, Osaka-shi Osaka 540-0022 (JP); KITANO, Kazuhito, Osaka-shi Osaka 540-0022 (JP)
(74) Representative: Müller-Boré & Partner Patentanwälte PartG mbB
(86) International application number: PCT/JP2009/064777
(87) International publication number: WO 2010/024244

(56) References cited:
- WO-A2-2006/119256
- JP-A- H05 509 024
- JP-A- H10 511 589
- JP-A- 2009 089 900
- US-A- 3 961 629
- US-A- 5 466 231
- US-A- 5 827 215
- US-A1- 2001 025 155

## Description

### TECHNICAL FIELD

The present invention relates to an endoscope excluder that is used for excluding an organ other than a target organ in a surgery (organ to be operated on) and is particularly suitable for use in an endoscopic surgery.

### BACKGROUND ART

Generally, an abdominal region or a chest region has been widely incised in a conventional surgical operation. However, due to the developments of medical technology and medical equipment, endoscopic surgeries are often executed in recent years. In such an endoscopic surgery, there are opened tiny holes at several positions in an abdominal region or the like, trocars (also called as trockars) are inserted into these holes, and a camera or a surgical instrument in an elongate shape (such as an endoscopic forceps or an endoscopic knife) are inserted through each of the trocars. The endoscopic surgeries are less invasive and apply low impact to patients.

Similarly to laparotomy (thoracotomy, or the like), an endoscopic surgery includes excision, extirpation, or the like of an organ. Such an operation may be possibly interrupted by an organ other than a target organ (an untargeted organ) and is thus difficult to be performed. Therefore desired is that such an untargeted organ interrupting a view is excluded to secure a visual field for the target organ.

For example, in a laparoscopic rectal surgery, because the small intestine falls into the cavity between the pelvis, the visual field for the rectum as the target organ is hard to be secured. In order to avoid such a situation, currently, the patient is laid in a posture with the head located at a low position to displace the small intestine toward a side of the head in the abdominal cavity. However, if the head is located extremely low, the hemodynamics may be affected or the brachial plexus may be excluded to possibly cause a thoracic outlet syndrome.

In this regard, consideration has been taken on the use of an excluder in order to lay a patient horizontal as much as possible. There has been proposed an excluding instrument (excluder) for the endoscopic surgeries, which is inserted into a body cavity through a trocar and is expanded in the body cavity to exclude an untargeted organ.

For example, Patent Document 1 describes an endoscope excluder including a rubber sheet, and a ring frame made of a super-elastic alloy and attached to surround the rubber sheet. In use thereof, this endoscope excluder is flattened out into a thin and straight shape, is inserted through a trocar, and is then expanded in width in a body cavity by a restoring force of the ring frame to exclude an organ.

Patent Document 2 describes an excluder (a support forceps) provided with a balloon at a tip end thereof. This excluder is inserted into a body cavity through a trocar with the balloon being folded and accommodated in a cylindrical haft. The haft is then slid to expose the balloon, into which gas is injected, so that the expanded balloon excludes or supports an organ. After the use thereof, the balloon is degassed and folded to be accommodated in the cylindrical shaft, and the excluder is removed from the trocar.
US 2001/0025155 A1 discloses the features of the preamble of claim 1.

### PRIOR ART DOCUMENTS

### PATENT DOCUMENTS

Patent Document 1: Japanese Unexamined Patent Publication No. 2003-164459
Patent Document 2: Japanese Unexamined Patent Publication No. 2005-253916

### SUMMARY OF THE INVENTION

### PROBLEMS TO BE SOLVED BY THE INVENTION

However, with any one of the conventional excluders described above, a surgeon or an assistant is required to hold a grip of the excluder to keep the excluding state during the surgery, thereby additional manpower is needed. Moreover, there is another problem that an additional hole used for supporting the excluder needs to be opened in the body of a patient.

Although each of the excluders described in Patent Documents 1 and 2 is configured to exclude an organ with use of the rubber sheet or the balloon, which are relatively soft, the frame or the haft to support the rubber sheet or the balloon is solid. In this regard, what is expected is an excluder less harmful to an organ in a body cavity.

The present invention focused on the circumstances described above, and it is an object thereof to provide an endoscope excluder that requires neither manpower for supporting the excluder nor a hole used for supporting the excluder to be opened in a body of a patient, as well as is capable of gently excluding an organ in a body cavity.

### SOLUTIONS TO THE PROBLEMS

The invention is defined in claim 1. It is noted that the expression "obtained by drying and compression molding" refers to a case of compressing and drying at one time by performing compression molding while drying, as well as a case of initially compressing and then drying in such a compressed state. Hereinafter, drying and performing compression molding may be occasionally referred to as "drying and compression molding". Further, the "hygroscopically expanding material" has a property of expanding by absorption of moisture.

As described above, in the endoscope excluder of the present invention, the excluder body has the transverse section smaller than the section of the cylindrical inner cavity of the trocar. Accordingly, the excluder can be inserted into a body cavity through the cylindrical portion of the trocar. Further, the excluder body is made of the hygroscopically expanding material obtained by drying and compression molding. Therefore, the excluder body is swollen to be increased in size when moisture is applied thereto.

The excluder body smaller than the section of the cylindrical inner cavity of the trocar is not large enough to exclude an organ. However, if physiological saline or the like is poured on the excluder body after the endoscope excluder is inserted into a body cavity, the excluder body is swollen and increased in size so as to exclude an organ. Moreover, because the hygroscopically expanding material having been swollen has an appropriate repulsive force, an untargeted organ can be excluded by the excluder body that is made of the hygroscopically expanding material having been swollen. Furthermore, because the hygroscopically expanding material having been swollen is soft, the excluder body, which is made of the hygroscopically expanding material having been swollen, is freely bendable and easily handled, and is capable of gently excluding an untargeted organ.

The above endoscope excluder of the present invention does not need manpower for supporting the excluder to keep the excluding state, or there is no need to provide an additional hole for the excluder in the body of a patient, unlike the endoscope excluders described in Patent Documents 1 and 2. Further, the endoscope excluder of the present invention does not have a hard frame portion, so that the swollen excluder body in the present invention is capable of gently excluding an untargeted organ.

The "transverse section" is in a direction perpendicular to the longitudinal direction.

The above expression "the excluder body has a transverse section smaller than a section of a cylindrical inner cavity of a trocar" specifically means that, in an exemplary case where the section of the cylindrical inner cavity of the trocar has a perfect circle shape and the excluder body has a bar shape and a section in a rectangular or square shape (the excluder body in a quadrangular prism shape), diagonal lines of the transverse section of the excluder body are shorter than a diameter of the section of the cylindrical inner cavity of the trocar. This expression "shorter" refers to a case of being substantially shorter. Even in a case where the excluder body having been dried and compressed is sized to be smaller than the section of the cylindrical inner cavity of the trocar, the excluder body may possibly absorb moisture in the air to be slightly expanded, which will result in a state where the diagonal lines of the transverse section of the excluder body are each as long as the diameter of the section of the cylindrical inner cavity of the trocar. However, even in such a case, if the excluder body is made of a slightly flexible material in a dried and compressed state, the excluder body can be inserted through the trocar while the surfaces of the excluder body are in contact with the inner wall surface of the trocar. Accordingly, such an excluder body can be still expressed as "shorter". Therefore, the present invention also includes a case where the excluder body slightly expanded is as large as the section of the cylindrical inner cavity of the trocar.

Further, the above expression "the excluder body has a transverse section smaller than a section of a cylindrical inner cavity of a trocar" means that the transverse section at any position in the longitudinal direction of the excluder body is smaller than the section of the cylindrical inner cavity of the trocar. In other words, the excluder body does not have a portion protruding outward. Such a portion protruding outward will be stuck and inhibits the excluder body from being inserted through the trocar.

In a laparotomy, there is a known measure of excluding an untargeted organ by displacing the organ with use of a thick sponge in a flat plate shape. This sponge excluder provided with no hard frame portion is capable of excluding an untargeted organ because the sponge itself exerts an appropriate repulsive force. Moreover, the sponge excluder is soft and is advantageously capable of excluding an untargeted organ more gently. The excluder does not need to be supported in order to keep the excluding state, which results in no need for supporting manpower. There is also proposed one of such sponge excluders for laparotomy, which is dried and compressed beforehand in order to reduce the storage space thereof, and is swollen by physiological saline or the like applied thereonto upon the use thereof.

However, this sponge excluder for laparotomy is large (260 mm x 90 mm x 35 mm or 120 mm x 90 mm x 35 mm, for example). Accordingly, this sponge excluder cannot be inserted through the cylindrical portion of a thin trocar. However, the excluder is required to have a certain size for exclusion. No organ can be excluded by a sponge excluder that is thin enough to pass through the cylindrical portion of the trocar.

In this regard, the endoscope excluder of the present invention, which is thin as described above, can be inserted through the cylindrical portion of the trocar. Moreover, because the excluder is sufficiently dried and compressed, the excluder can be swollen and increased in size. Therefore, an organ can be excluded by the excluder that is swollen and increased in size.

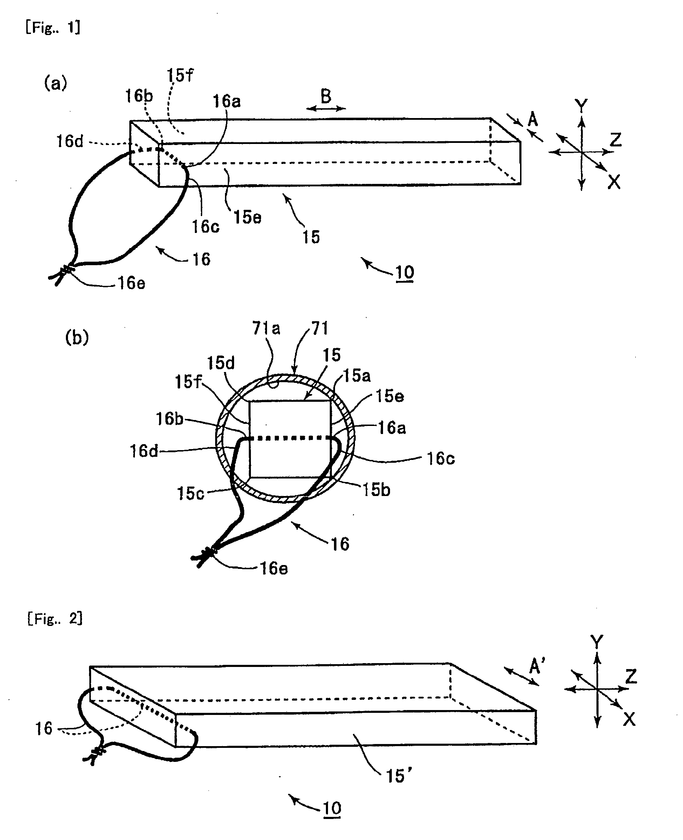

As described above, the excluder body in the bar shape according to the present invention is obtained by drying and compression molding. In this case, the excluder body is compressed at least from sides of the bar (from opposing sides intersecting with the longitudinal direction). In other words, in the present invention, the excluder body in the bar shape has a long axis that is defined to be aligned with a Z axis, and the hygroscopically expanding material is compressed in directions perpendicular to the Z axis(see an arrow A in Figs.1(a) described below, for example). In this configuration, when moisture is applied, the excluder body is swollen and expanded in the directions of the sides of the bar (in the directions of increasing the width of the bar). Therefore, even if the transverse section of the excluder body in the dried and compressed state is smaller than the cylindrical inner cavity of the trocar, the excluder body is changed into a plate shape and is capable of easily excluding an organ when being swollen after having been inserted into a body cavity.

In present invention, the endoscope excluder further comprises a long frame that accommodates the excluder body so as to be taken out thereof, wherein sides perpendicular to the compression directions of the excluder body are preferably provided with paired support walls facing each other along the Z axis. More specifically, the endoscope excluder is provided with the frame, which preferably has the support walls on the compressed surfaces of the excluder body. In the following description, such an endoscope excluder provided with a frame is referred to as a "framed excluder", while an excluder provided with no frame is simply referred to as an "endoscope excluder" or an "excluder", for the purpose of distinction therebetween.

As described above, the excluder body is swollen and increased in size when moisture is applied thereto. Due to this property, the excluder before use may be slightly expanded by absorbing ambient moisture at the stage of sterilization treatment or storage. If the excluder body is expanded too much, the excluder body may be grown to be larger than the cylindrical inner cavity of the trocar and may not be inserted through the trocar.

In this regard, if the excluder body is accommodated in the frame as described above, the expansion can be suppressed to keep the size of the excluder body so as to be inserted through the cylindrical inner cavity of the trocar.

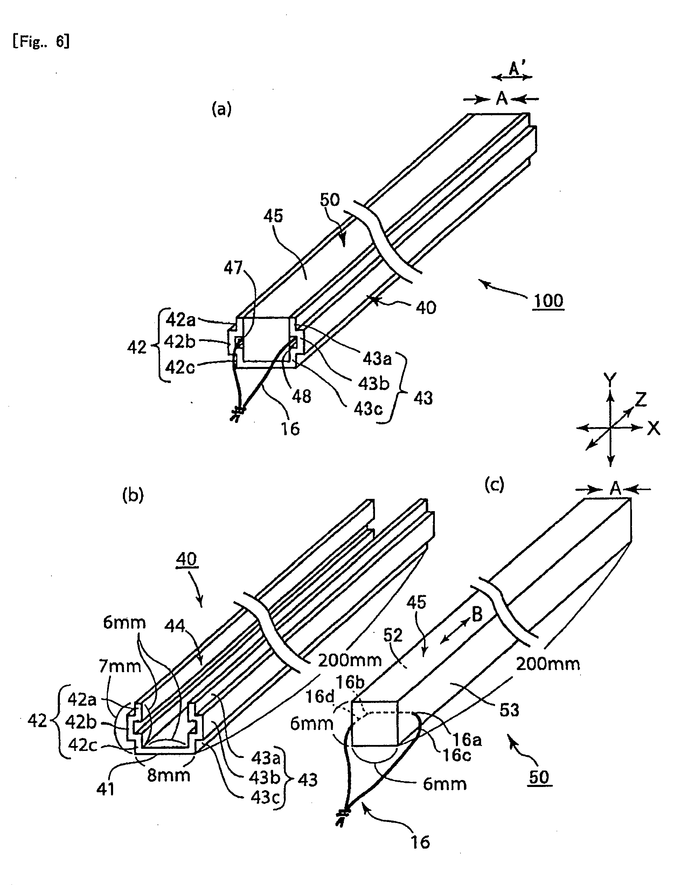

The excluder body is expanded only in the directions opposite to the compression directions during the manufacture of the excluder body. Therefore, the frame only needs to have the support walls at least on the compressed sides of the excluder body. Moreover, the support walls of the frame need not be provided entirely on the compressed sides of the excluder body, but have only to be provided to a degree of being capable of inhibiting the expansion. For example, as shown in Fig. 6(a), the frame may be provided with grooves at the centers of the sides corresponding to the compressed surfaces of the excluder body, so that regions above and below the grooves can support the excluder body (see left and right side walls 42 and 43 of a frame 40).

In the framed excluder, the frame preferably has an opened portion on a side other than the sides perpendicular to the compression directions.

When the framed excluder is processed by gas sterilization, the opened portion provided as described above causes the endoscope excluder to be sufficiently exposed to sterilization gas (such as ethylene oxide gas), so that the sterilization treatment can be preferably performed. However, if the opened portion is provided on one of "the sides perpendicular to the compression directions", the excluder body may be expanded in the opened portion. Therefore, as described above, the opened portion is preferably provided on a side other than "the sides perpendicular to the compression directions".

In the framed excluder , when two directions perpendicular to the Z axis are defined to be along an X axis and a Y axis, the excluder body is compressed along the X axis, and the frame is preferably opened at least on one side in the direction along the Y axis(the direction intersecting with the compression directions of the excluder body).

In such a case where the frame is opened on one side in the Y axis, similarly to the above, the endoscope excluder can be sufficiently exposed to sterilization gas (such as ethylene oxide gas) to excellently perform the gas sterilization treatment.

In view of the original purpose of preventing the expansion of the excluder body, the opened side is preferably in the direction (along the Y axis) intersecting with the compression directions (along the X axis) of the excluder body as described above. In this case, only one side in the direction along the Y axis may be opened, or both sides in the direction along the Y axis may be opened.

In the framed excluder, the frame has a bottom wall on another side in the direction along the Y axis, and a tongue portion at least partially on an inner wall surface of the bottom wall so as to be along the inner wall surface, and the tongue portion protrudes from the bottom wall in one direction along the Z axis so as to be preferably raised toward the open side in the direction along the Y axis.

In this framed excluder, the excluder body is accommodated in a space surrounded by the bottom wall, the tongue portion, and the support walls. The excluder body is lifted up when the tongue portion is raised. The entire excluder body may be taken out of the frame by pinching the portion lifted up with fingers. In this manner, in the above framed excluder, the excluder body accommodated in the frame can be easily taken out thereof.

In the endoscope excluder according to the present invention, the excluder body absorbs moisture to be expanded preferably at an expansion factor of five times or more in the compression directions, and more preferably ten times or more. With such an expansion factor, the excluder body is expanded and sufficiently increased in size so as to preferably exclude an organ.

The excluder body preferably has a compressibility of 20% or less, and more preferably 10% or less. Such a compressibility will lead to excellent expansion by the application of moisture. The compressibility (%) is calculated by "the size in the compression directions after drying and compression molding" / "the size in the same directions when manufactured without compression" x 100.

In present invention, the hygroscopically expanding material is preferably a cellulose sponge. It is because the cellulose sponge is excellent in view of safety to a living body. Moreover, the cellulose sponge is a material that can be obtained by drying and compression molding. When moisture is applied, the dried and compressed cellulose sponge absorbs moisture to be expanded, thereby being regarded as a preferable material on this point.

In present invention, the excluder body in the bar shape is cut so as to have a longitudinal direction(along the Z axis) thereof that is aligned with a direction of extruding the cellulose sponge in a production process thereof, and when the extrusion direction is defined to be along a Z axis and two directions perpendicular to the extrusion direction are defined to be along an X axis and a Y axis, the cellulose sponge is compressed in the directions along the X axis and/or the Y axis.

The cellulose sponge is generally produced by extruding a raw material from a nozzle into a mold to solidify the same. This production method is likely to cause a fiber ingredient to be aligned with the extrusion direction. Accordingly, the cellulose sponge is easily split in the extrusion direction rather than a different direction. Therefore, the excluder body in the bar shape is cut such that the extrusion direction is perpendicular to the longitudinal direction, such an excluder body in the bar shape tends to be bent and divided. In this regard, in a case where the excluder body in the bar shape is cut such that the extrusion direction is aligned with the longitudinal direction (along the Z axis), the excluder body is less likely to be split and divided.

Moreover, the fiber ingredient in the cellulose sponge tends to be aligned with the extrusion direction in the production process as described above, the sponge can be compressed further easily in the directions (along the X axis and/or the Y axis) intersecting with the extrusion direction. The compressibility is preferably 20% or less, and more preferably 10% or less. Such a compressibility will lead to excellent expansion by the application of moisture.

In the endoscope excluder according to the present invention, the excluder body has a contrast thread inserted therethrough and knotted, the excluder body has, on side surfaces along a longitudinal direction, two positions in total each provided with a flat portion or a concave portion, and the contrast thread is preferably inserted through the excluder body via the flat portion or the concave portion. The expression "the excluder body has a contrast thread inserted therethrough and knotted" means that the contrast thread is inserted through the excluder body and is knotted.

As described above, the endoscope excluder according to the present invention is advantageous as requiring no manpower to support the excluder. However, this feature leads to a danger of losing the excluder in a body cavity. In a case where the inserted excluder remains in the body cavity, there are risks of causing various bad physical conditions such as pain, discomfort, and fever. Accordingly, the excluder needs to be removed from the body upon completion of a surgery. However, the excluder body having blood or the like adhering thereto is hard to be megascopically found.

Regarding this problem, there is adopted a measure in which a hygiene material such as gauze has a contrast thread attached thereto, and radiographic visualization (X-ray photography) is performed at the final stage of the surgery to check existence of the gauze by looking for the contrast thread. In this method, the gauze is removed if remaining in the body. In this case, generally adopted as the contrast thread is a line of thread made of a roentgenopaque resin. It is noted that the sponge excluder for laparotomy described above is provided with a hole through which gauze having a strip shape and a contrast thread is inserted therethrough and attached thereto.

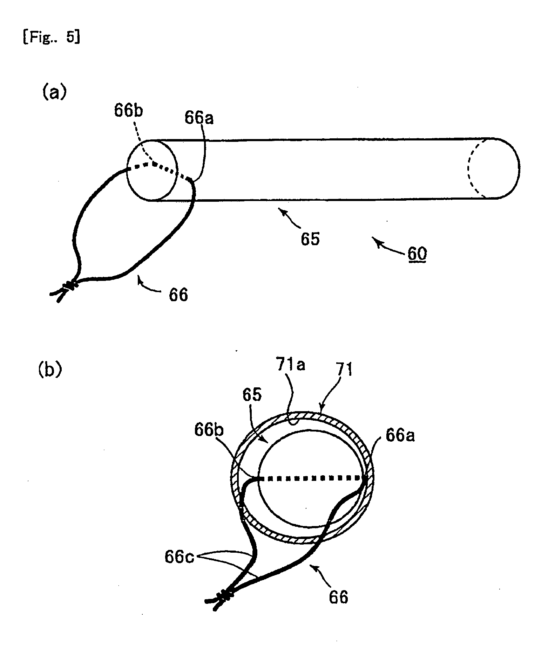

Nevertheless, there still arises the following problem even if the method used for gauze is applied to the endoscope excluder. More specifically, it will be possible to attach the contrast thread by inserting through and knotting for the excluder body made of a hygroscopically expanding material that is obtained by drying and compression molding. However, as shown in Figs. 5(a) and 5(b) for example, in a case where a excluder body 65 has a columnar shape (Fig. 5(a) which is a perspective view of an endoscope excluder 60 including an the excluder body 65 in the columnar shape through which a contrast thread 66 is inserted and knotted), entering/exiting portions 66a and 66b of the inserted contrast thread 66 may be stuck in a trocar cylindrical inner wall 71a and the contrast thread 66 may be frictioned and cut off (Fig. 5(b) which is a sectional view showing a state where the endoscope excluder 60 passes through a cylindrical portion of a trocar 71).

It is noted that, in a contrast thread portion 66c distant from the entering/exiting portions 66a and 66b of the inserted contrast thread, the contrast thread 66 can be easily prevented from being frictioned with the trocar cylindrical inner wall 71a.

If the endoscope excluder 60 is carefully inserted so as to pass through the trocar 71 in the center of the cylindrical inner cavity, the entering/exiting portions 66a and 66b of the inserted contrast thread will not be stuck in the trocar cylindrical inner wall 71 and the contrast thread 66 will not be cut off. However, such a manner of insertion unrealistically requires high skill to a surgeon.

This problem is solved as described above in the present invention such that the excluder body in the bar shape is provided on each of the side surfaces with the flat portion or the concave portion so as to allow the contrast thread to be inserted therethrough. More specifically, the cylindrical inner cavity of a trocar generally has a perfect circle shape or an elliptical shape, while a trocar in a rectangular shape is not currently used. The flat portion or the concave portion of the excluder body is not in contact with the inner wall surface of the trocar in such an arc shape but there is formed a gap therebetween. In the present invention, the inlet and the outlet for inserting through the contrast thread are located in the gaps. Therefore, the contrast thread is not frictioned with the cylindrical inner wall of the trocar and is thus quite unlikely to be cut off.

Each of the inlet and the outlet for inserting through the contrast thread is preferably located at an approximate center in the thickness direction in the flat portion or the concave portion. In such a case where the inlet and the outlet are each located at the approximate center, the gap is increased in size.

The flat portion or the concave portion needs not to be provided throughout the longitudinal length of the excluder body, but may be formed at least at one end of the excluder body in the bar shape.

In this manner, the contrast thread can be inserted, without being cut off, into a body cavity together with the excluder body. Therefore, it is possible to check whether or not the endoscope excluder remains in the body by looking for the contrast thread through radiographic visualization.

In the present invention, the hygroscopically expanding material is a cellulose sponge, and when an extrusion direction in a process of producing the cellulose sponge is defined to be along a Z axis and two directions perpendicular to the extrusion direction are defined to be along an X axis and a Y axis, the contrast thread is preferably inserted through the cellulose sponge along the X axis or the Y axis(the direction intersecting with the extrusion direction in the process of producing).

As described above, the cellulose sponge is further easily split in the extrusion direction in the production process thereof. Accordingly, if the contrast thread is inserted in the extrusion direction (along the Z axis), the cellulose sponge is likely to be split. However, when the contrast thread is inserted in the direction (along the X axis or the Y axis) intersecting with the extrusion direction as described above, the cellulose sponge is unlikely to be split.

Moreover, in the present invention, the cellulose sponge is compressed along the X axis in the process of producing the cellulose sponge, and the contrast thread is preferably inserted through the cellulose sponge along the X axis and knotted.

In the case where the contrast thread is inserted in the compression directions (along the X axis) of the cellulose sponge, the length of the contrast thread passing through the cellulose sponge is increased when moisture is applied and the sponge is swollen. Accordingly, the contrast thread is further unlikely to fall off.

In the framed excluder according to the present invention, the frame is preferably provided with a groove or a gap along the support wall.

In one possible method of using the framed excluder, the tip end of the framed excluder is placed to face the insertion hole of the trocar, the endoscope excluder in the frame (the excluder body having the contrast thread attached thereto) is pushed from the rear end so as to be extracted from the frame and inserted into the trocar (during this work, the positional relationship does not change between the frame and the trocar). In this case, there will be a risk that the contrast thread is stuck between the excluder body and the inner wall surface of the frame. However, in such a case where there is adopted the frame provided with the longitudinal groove (or the gap) as described above and the endoscope excluder is accommodated in the frame, if the groove (or the space) in the frame is located at the inlet and the outlet for the inserted contrast thread (in other words, if the contrast thread is inserted through the excluder body so as to be located at the position of the groove (or the gap)), the entering/exiting portions of the inserted contrast thread moves along the groove while the endoscope excluder is being extracted. Therefore, the contrast thread is quite unlikely to be stuck or frictioned.

In the endoscope excluder according to the present invention, the excluder body preferably has a roentgenopaque resin attached thereto by thermal fusion bonding. More specifically, the excluder body may have, in place of the contrast thread, such a roentgenopaque resin attached thereto by thermal fusion bonding (hereinafter, this attached portion by thermal fusion bonding may be referred to as a contrast portion). It is possible to check existence of the endoscope excluder by looking for the contrast portion through radiographic visualization (X-ray photography).

### EFFECTS OF THE INVENTION

In an endoscope excluder according to the present invention, an excluder body is obtained by drying and compression molding and has a bar shape with a transverse section being smaller than a section in a cylindrical inner cavity of a trocar. Therefore, the excluder body can be inserted into a body cavity through the trocar. The excluder body is expanded in the body cavity so as to gently exclude an organ while securing a view to a target organ. Moreover, the present invention requires neither manpower for supporting the excluder nor a hole to be opened in a body of a patient in order to support the excluder.

Further, in a framed excluder according to the present invention, an excluder body can be inhibited from expanding even in a case of being exposed to moisture during the sterilization treatment, storage, or the like. Therefore, the excluder body can be kept in size so as to be inserted through the cylindrical portion of the trocar.

Moreover, in the present invention, in an endoscope excluder including an excluder body that has a contrast thread inserted therethrough, the contrast thread is inserted in a flat portion or a concave portion on each of side surfaces of the excluder body. Therefore, the contrast thread is less likely to be cut off when the endoscope excluder is inserted into a body cavity through a trocar.

### BRIEF DESCRIPTION OF THE DRAWINGS

Fig. 1(a) is a perspective view of an endoscope excluder according to a first embodiment of the present invention, and Fig. 1(b) is a sectional view showing a state where the endoscope excluder passes through a cylindrical portion of a trocar in the first embodiment.
Fig. 2 is a perspective view showing a state where an excluder body of the endoscope excluder has been swollen in the first embodiment.
Fig. 3 is a perspective view of an endoscope excluder according to a second embodiment of the present invention.
Fig. 4 is a perspective view of an endoscope excluder according to a third embodiment of the present invention.
Fig. 5(a) is a perspective view of an endoscope excluder including an excluder body in a columnar shape through which a contrast thread is inserted and knotted, and Fig. 5(b) is a sectional view showing a state where the endoscope excluder passes through a cylindrical portion of a trocar.
Fig. 6(a) is a perspective view of a framed excluder according to a fourth embodiment of the present invention, Fig. 6(b) is a perspective view of such a frame, and Fig. 6(c) is a perspective view of such an endoscope excluder.
Fig. 7 is a front view of the frame according to the fourth embodiment.
Fig. 8 is a perspective view showing a state where the framed excluder is inserted into a trocar.
Fig. 9 is a perspective view of a framed excluder according to a fifth embodiment of the present invention.
Fig. 10(a) is a perspective view of a framed excluder according to a sixth embodiment of the present invention, Fig. 10(b) is a perspective view of such a frame, and Fig. 10(c) is a perspective view of an endoscope excluder (an excluder body and a contrast portion) therefor.
Fig. 11 is a side view of the framed excluder according to the sixth embodiment in a state where the endoscope excluder is taken out of the frame.

### MODES FOR CARRYING OUT THE INVENTION

### First Embodiment

Fig. 1(a) is a perspective view of an endoscope excluder 10 according to a first embodiment of the present invention. Fig. 1(b) is a sectional view showing a state where the endoscope excluder 10 passes through a cylindrical portion of a trocar 71 in the first embodiment. Fig. 2 is a perspective view showing a state where an excluder body 15 of the endoscope excluder 10 has been swollen (an excluder body 15') in the first embodiment.

The excluder 10 includes the excluder body 15 and a contrast thread 16. The excluder body 15 is made of a cellulose sponge and has a bar shape with a square shape in section (200 mm x 8 mm x 8 mm), of which longitudinal direction (along a Z axis) is aligned with an extrusion direction (indicated by arrows B) in a process of producing the cellulose sponge (it is noted that the longitudinal length thereof is shorter than the actual size for convenience in the figures). The excluder body 15 is compressed at a compressibility of 10% from side surfaces of the bar, that is, in directions (indicated by arrows A along an X axis) intersecting with the longitudinal direction (indicated by the arrows B along the Z axis). The excluder body 15, which is sized as described above, can be inserted through the trocar having a cylindrical inner cavity in section (in a perfect circle shape) with a diameter of 12 mm.

The cellulose sponge can be produced by adopting a cellulose regeneration method or a cellulose solvent solution method. For example, such a cellulose sponge is disclosed in Japanese Patent No. 3520511.

Specifically exemplified is a method for manufacturing the excluder body 15. Initially, viscose is obtained from a cellulosic material such as wood chips. Natural fibers are added to the viscose, and further mixed thereinto are crystallized mirabilite, a polyol compound, and a surfactant if appropriate. Sponge liquid concentrate thus obtained is extruded and filled in a mold tool to be heated and solidified. This solidified sponge is cut into a thick strip shape (200 mm x 8 mm x 80 mm) such that the extrusion direction is aligned with a longitudinal direction thereof (along the Z axis) (it is noted that the extrusion direction is aligned with the arrows B indicated in Fig. 1(a)). This strip is dried while being compressed in directions perpendicular to the longitudinal direction (it is noted that such compression directions are aligned with the arrows A indicated in Fig. 1(a)), so as to obtain the excluder body 15 in a thin and long bar shape (200 mm x 8 mm x 8 mm) as described above.

The contrast thread 16 is a line of thread made of a roentgenopaque resin, and specific examples thereof include a multifilament obtained by kneading barium sulfate into a polypropylene series resin, and a filum obtained by kneading barium sulfate into a vinyl chloride resin or a silicon series resin.

The contrast thread 16 is inserted through the excluder body 15 in the bar shape near one end thereof along the compression directions (indicated by the arrows A along the X axis) and is knotted (a knotted portion 16e) into a ring shape. The contrast thread 16 enters and exits the excluder body 15 at centers in the thickness direction (along a Y axis) of flat portions 15e and 15f on the side surfaces. The contrast thread 16 is relatively long in consideration of the state where the excluder body 15 is swollen (the excluder body 15') (see Fig. 2).

Described below is a method of using the excluder 10 according to the present first embodiment in a laparoscopic rectal surgery as an example.

A patient is temporarily laid in a posture with the head located at a low position to displace the small intestine upward due to the gravity. The excluder 10 is inserted into the abdominal cavity through a cylindrical portion of the trocar inserted in the abdominal region from the end not provided with the contrast thread 16. In this case, as shown in Fig. 1(b), the excluder body 15 is brought into contact with a trocar cylindrical inner wall 71a at corners 15a, 15b, 15c, and 15d, and there are formed gaps at an inlet and an outlet for the contrast thread 16 in the flat portions 15e and 15f. Accordingly, entering/exiting portions 16a and 16b of the inserted contrast thread are not frictioned with the trocar cylindrical inner wall 71a. Because portions 16c and 16d extending respectively from the entering/exiting portions 16a and 16b of the inserted contrast thread are drawn along the flat portions 15e and 16f, the portions 16c and 16d are not stuck in the trocar cylindrical inner wall 71a. Therefore, the contrast thread 16 is less likely to be cut off.

The excluder body 15 is then disposed near the root of the ligament of the small intestine that was displaced upward, and physiological saline is poured thereon. As shown in Fig. 2, the compressed excluder body is swollen in directions indicated by arrows A' to be restored and increased in size (the excluder body 15', an expansion factor: ten times). The small intestine is excluded by the excluder 10 thus swollen (approximately 200 mm x 8 mm x 80 mm), so that a view to the target organ is secured even when the posture of the patient is returned to the horizontal state.

As described above, with use of the excluder 10 according to the present embodiment, the patient is laid in a posture with the head located at a low position only at the initial stage of the surgery unlike in a conventional surgery in which a patient needs to be laid in a posture with the head located at a low position throughout the surgery. Accordingly, the hemodynamics is not affected by the posture with the head at a low position, and a complication such as a thoracic outlet syndrome can be also prevented.

In a conventional laparoscopic surgery, the surgery is usually executed in an extreme low head posture (Trendelenburg posture) because there is no effective excluder for excluding an organ such as the small intestine out of the pelves. There is an academic report on securing a view to a target organ by nasally inserting a catheter provided at a tip end with a balloon three or four days before the surgery and handling the catheter to exclude the small intestine out of the pelves. These measures are all seriously harmful to a patient. However, the use of the excluder 10 according to the present invention enables appropriate exclusion of an organ to secure a view without adopting any one of these measures.

When the treatment of the target organ is completed and the exclusion is no more required, the excluder 10 of the present invention is taken out of the body through a small incised part that is opened in the abdominal region and is used to take out the excised organ (the small incised part used for taking out the excised target organ (a small incised wound)). In a case where the excluder 10 is hard to be visually recognized, it is checked whether or not the excluder 10 remains in the body by looking for the contrast thread 16 to be imaged by radiographic visualization. If remaining in the body, the excluder is taken out of the body.

There may be an idea that the endoscope excluder is not necessarily sized so as to be inserted through the cylindrical portion of the trocar if the small incised part is formed at the initial stage of the surgery to insert the endoscope excluder into the body cavity. However, such a small incised part is usually formed at the final stage of a surgery, so that it is desired to avoid the formation of the small incised part at the initial stage of the surgery only for the purpose of inserting the endoscope excluder.

As described above, when using the present invention, the small incised part is used to take out the endoscope excluder, and the endoscope excluder is inserted into the body cavity through the trocar.

### Second Embodiment

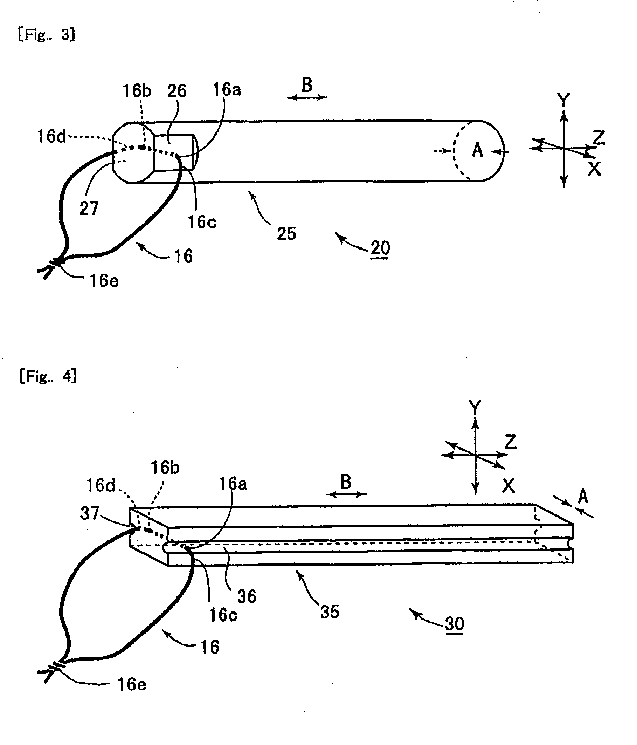

Fig. 3 is a perspective view of an endoscope excluder 20 according to a second embodiment of the present invention.

The excluder 20 includes an excluder body 25 that has an approximately columnar shape in its entirety and has a transverse section smaller than a section of the cylindrical inner cavity of the trocar. The excluder body 25 is notched from one end to a vicinity thereof so as to provide flat portions 26 and 27. These two flat portions 26 and 27 are provided so as to face each other in the compression directions (indicated by arrows A along the X axis) in the process of manufacturing the excluder body 25. In other words, the two flat portions 26 and 27 are provided on the excluder body so as to be symmetrical with respect to a point on the axis of the excluder body in a bar shape. There are provided an inlet and an outlet for a contrast thread 16 approximately at centers of these flat portions 26 and 27. The excluder is configured identically to that of the first embodiment in the other portions.

Similarly to the first embodiment, the excluder 20 of the present second embodiment can be inserted into a body cavity through a trocar. When the excluder is swollen to be increased in size in the body cavity so as to exclude an organ when physiological saline or the like is poured thereon.

When being inserted through the trocar, the entering/exiting portions 16a and 16b of the inserted contrast thread 16 are distant from the cylindrical inner wall of the trocar. Therefore, the contrast thread 16 is seldom frictioned to be cut off. Further, the flat portions 26 and 27 are provided so as to reach one end of the excluder body 25. If the portions 16c and 16d extending respectively from the entering/exiting portions 16a and 16b of the inserted contrast thread are drawn along the flat portions 26 and 27, the portions 16c and 16d are not stuck in the cylindrical inner wall of the trocar. Therefore, the contrast thread 16 is less likely to be cut off.

### Third Embodiment

Fig. 4 is a perspective view of an endoscope excluder 30 according to a third embodiment of the present invention.

The excluder 30 includes an excluder body 35 that has an approximately quadrangular prism shape in its entirety and has a transverse section smaller than a section of the cylindrical inner cavity of the trocar. The excluder body 35 is provided with concave portions 36 and 37 respectively on surfaces in the compression directions (indicated by arrows A along the X axis) in the process of manufacturing the excluder body 35. The contrast thread 16 is inserted through the excluder body 35 into/from an inlet and an outlet in the concave portions 36 and 37 in the vicinity of one end of the excluder body 35. The excluder is configured identically to that of the first embodiment in the other portions.

Similarly to the first embodiment, the excluder 30 of the present third embodiment can be inserted into a body cavity through the trocar. The excluder is swollen to be increased in size in the body cavity so as to exclude an organ when physiological saline or the like is poured thereon.

When being inserted through the trocar, the entering/exiting portions 16a and 16b of the inserted contrast thread 16 are distant from the cylindrical inner wall of the trocar. Therefore, the contrast thread 16 is seldom frictioned to be cut off. Further, when the portions 16c and 16d extending respectively from the entering/exiting portions 16a and 16b of the inserted contrast thread are drawn so as to be away from corners of the quadrangular prism of the excluder body 35, the portions 16c and 16d are not stuck in the cylindrical inner wall of the trocar. Therefore, the contrast thread 16 is less likely to be cut off

### Fourth Embodiment

Fig. 6(a) is a perspective view of a framed excluder 100 according to a fourth embodiment of the present invention. Fig. 6(c) is a perspective view of an excluder 50 in the framed excluder 100. Fig. 6(b) is a perspective view of a frame 40 in the framed excluder 100. Fig, 7 is a front view of the frame 40. It is noted that the portions denoted by the symbols same as those in Figs. 1(a) and 1(b) are constituent portions same as those of the example shown in Figs. 1(a) and 1(b).

The endoscope excluder 50 includes an excluder body 45 and a contrast thread 16.

As shown in Fig. 6(c), the excluder body 45 in the excluder 50 has a bar shape with a square shape in transverse section (200 mm x 6 mm x 6 mm). This excluder body 45 is compressed from two side surfaces 52 and 53 that face each other out of four side surfaces (arrows A along the X axis). The excluder 50 is configured identically with the excluder 10 of the first embodiment in the other portions.

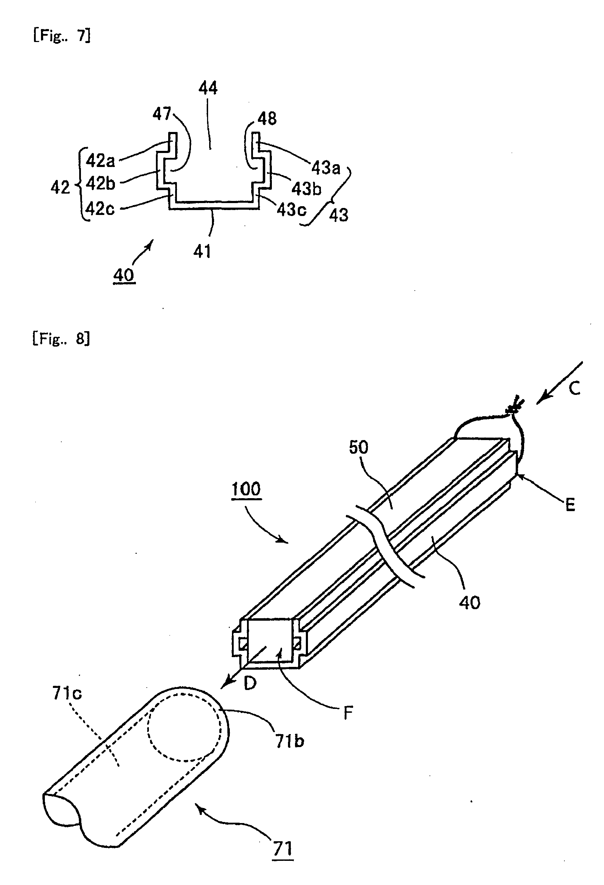

As shown in Fig. 6(b), the frame 40 has a long body and includes left and right side walls 42 and 43 linearly extending in the longitudinal direction (along the Z axis) as well as a bottom wall 41. The frame 40 has an inner cavity configured by a main cavity 44 in a square prism shape and two sub cavities 47 and 48 that are provided continuously from the main cavity (see also Fig. 7). The sub cavities 47 and 48 are spaces in grooves formed respectively in the side walls 42 and 43 of the frame 40. More specifically, the left and right side walls 42 and 43 are provided with recesses (grooves) 42b and 43b throughout in the longitudinal direction thereof. The sub cavities 47 and 48 are provided in the recesses 42b and 43b, respectively. The main cavity 44 is formed by the bottom wall 41 and support walls 42a, 42c, 43a, and 43c (left and right side wall portions other than the recesses 42b and 43b) in the left and right side walls 42 and 43. The main cavity 44 is as large as 200 mm x 6 mm x 6 mm so that the excluder body 45 is tightly fit therein as shown in Fig. 6(a).

As shown in Fig. 6(a), the framed excluder 100 includes the frame 40 and the excluder body 45 that is accommodated in the main cavity 44 of the frame 40. The excluder body 45 is supported in the compression directions (indicated by the arrows A along the X axis) by the support walls 42a, 42c, 43a, and 43c in the left and right side walls 42 and 43 of the frame 40. The bottom wall 41 is provided on one of the sides in the direction (along the Y axis) intersecting with the compression directions (indicated by the arrows A) of the excluder body 45, while the other side is open. In this manner, the excluder body 45 is exposed on one side thereof.

The entering/exiting portions 16a and 16b of the inserted contrast thread 16 are located in the sub cavities 48 and 47 that are configured by the recesses 43b and 42b in the right and left side walls 43 and 42, respectively. Moreover, the portions 16c and 16d extending from the entering/exiting portions 16a and 16b of the inserted contrast thread are also located in the sub cavities 48 and 47.

Upon performing gas sterilization or the like, the excluder body 45 tends to be expanded in directions (indicated by arrows A' along the X axis) opposite to the compression directions by moisture generated in the treatment process. However, with the framed excluder 100 described above, such expansion is suppressed by the support walls 42a and 42c and the support walls 43a and 43c facing each other, to keep the predetermined size (6 mm in width in the example shown in the figures). Expansion is suppressed also in storage even though the excluder body 45 is tends to be expanded due to moisture in the air.

As described earlier, the frame 40 is opened on one of the two sides in the direction (along the Y axis) intersecting with the compression directions of the excluder body 45. The excluder body 45 is thus exposed to the sterilization gas on this portion. Therefore, the effect of the gas sterilization can be surely obtained.

More specifically, the excluder body 45, which is made of a cellulose sponge, is sterilized by sterilization gas (such as ethylene oxide gas) that is osmosed into the excluder body 45 in the gas sterilization treatment. In a case where the frame surrounds the four sides of the excluder body 45, the sterilization gas flows only by way of upper and lower end surfaces (6 mm x 6 mm) of the excluder body 45 in the bar shape. In such a case, in order to sterilize the entire excluder body 45 in the bar shape with 200 mm in length as in the example shown in the figures, the sterilization gas is to reach the maximum depth 200 mm /2 = 100 mm. As a result, the excluder body needs to be exposed to the sterilization gas for quite a long period of time.

However, in such a case as in the fourth embodiment described above where the frame 40 is opened one one side, the sterilization gas is osmosed also from this open side (6 mm x 200 mm). Therefore, the sterilization gas is to reach the maximum depth of at most 6 mm, and the sterilization treatment can be surely achieved in a short period of time.

Sterilized in the gas sterilization treatment is the entire framed excluder 100, of course inclusive of the contrast thread 16 and the frame 40.

The gas sterilization treatment is recommended to be performed onto the framed excluder 100 that is accommodated in a package having permeability to gas. The framed excluder 100 is desirably supplied to a user while being accommodated in the package, so that package is opened to take the framed excluder 100 out immediately before the use thereof.

Described next is a method of using the framed excluder 100 in an endoscopic surgery. Fig. 8 is a perspective view showing a state where the framed excluder 100 is inserted into a trocar 70.

An end F of the framed excluder 100 not provided with the contrast thread 16 is placed to face an insert hole 71b of the trocar. The excluder 50 is then extruded in the longitudinal direction thereof (along the Z axis) (an arrow C) from an end E provided with the contrast thread 16 with use of a clean extrusion bar (not shown). Accordingly, the excluder 50 is extracted from the frame 40 (an arrow D) and is inserted into a cylindrical inner cavity 71c of the trocar 70.

In this state, the entering/exiting portions 16a and 16b as well as the portions 16c and 16d extending therefrom (see Fig. 6(c)) of the inserted contrast thread 16 of the excluder 50 moves in the sub cavities 48 and 47 (in the recesses 43b and 42b) that are provided in the longitudinal direction (see Fig. 6(a)). Therefore, neither the entering/exiting portions 16a and 16b nor the portions 16c and 16d of the inserted contrast thread are not stuck or frictioned between the excluder body 45 and the inner wall surfaces of the frame 40. The remaining portions (portions from the extending portions 16c and 16d to the knotted portion 16e) of the contrast thread 16 are located behind a rear end surface (the end face at the end E provided with the contrast thread 16) of the excluder body 45. Accordingly, these remaining portions are not stuck between the excluder body 45 and the inner wall surfaces of the frame 40 (see Figs. 6(a) and 8).

It is noted that the frame 40 does not enter the cylindrical inner cavity 71c of the trocar because the frame 40 is larger than the cylindrical inner cavity 71c of the trocar.

The excluder 50 thus having entered the cylindrical inner cavity 71c of the trocar 70 is inserted into a body cavity through the trocar 70, and physiological saline is poured thereon so that the excluder is swollen to exclude an organ, similarly to the first embodiment.

### Fifth Embodiment

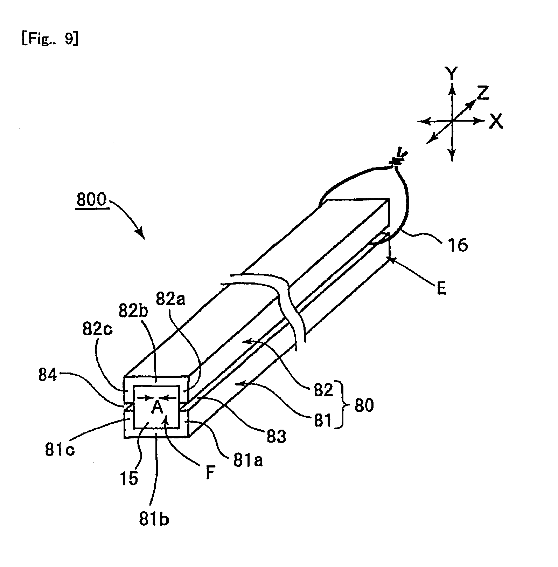

Fig. 9 is a perspective view of a framed excluder 800 according to a fifth embodiment of the present invention. It is noted that the portions denoted by the symbols same as those in Figs. 1(a) and 1(b) are constituent portions same as those of the example shown in Figs. 1(a) and 1(b).

The framed excluder 800 according to the fifth embodiment includes the excluder body 15 provided with the contrast thread 16 and a frame 80 accommodating the excluder body 15. The frame 80 has an upper member 82 and a lower member 81, and the upper member 82 and the lower member 81 can be separated from each other. In other words, the frame 80 can be divided into two portions along the longitudinal direction.

The upper member 82 and the lower member 81 each have a cornered U-letter shape in section. More specifically, the upper member 82 is provided with a top surface 82b and support walls 82a and 82c each standing on the top surface 82b, and the lower member 81 is provided with a bottom surface 81b and support walls 81a and 81c each standing on the bottom surface 81b. The support walls 82a and 82c of the upper member 82 and the support walls 81a and 81c of the lower member 81 support the excluder body 15 in the compression directions (arrows A along the X axis).

There are formed gaps 83 and 84 between the upper member 82 and the lower member 81 in a state where these members are attached to the excluder body 15. The entering/exiting portions (the entering/exiting portions of the inserted contrast thread 16 with respect to the excluder body 15) of the contrast thread 16 are located in the gaps 83 and 84 so that the contrast thread 16 is not stuck.

The excluder body 15 is expanded only in the directions opposite to the compression directions (the arrows A) of the excluder body in the manufacturing process thereof. The expansion of the excluder body 15 can be suppressed also in the present fifth embodiment because the frame 80 is provided with the support walls 82a, 82c, 81a, and 81c in the compression directions of the excluder body 15. Therefore, the excluder body can be kept in size so as to be inserted through the cylindrical inner cavity of the trocar.

As described above, the gaps (opened portions) 83 and 84 are formed throughout the longitudinal length (along the Z axis) of the frame 80. Accordingly, sterilization gas is osmosed into the excluder body 15 from the gaps 83 and 84 in the gas sterilization treatment, which shortens the period of time required for the sterilization treatment.

Upon use of the framed excluder 800 according to the fifth embodiment, the upper member 82 and the lower member 81 are separated from each other in departing directions (upward and downward directions in the figure), and the excluder (the excluder body 15 provided with contrast thread 16) inside is taken out. The excluder is then inserted into a body cavity through the cylindrical portion of the trocar, and physiological saline is poured thereon so that the excluder is swollen to exclude an organ, as having been described earlier.

Again in the present fifth embodiment, the end F of the framed excluder 800 not provided with the contrast thread 16 may be placed to face the insert hole of the trocar. Then, the excluder (the excluder body 15 and the contrast thread 16) may be inserted into the cylindrical portion of the trocar while being extruded in the longitudinal direction thereof (along the Z axis). In this case, the contrast thread 16 moves along the gaps 83 and 84 in the longitudinal direction so as not to be stuck or frictioned.

### Sixth Embodiment

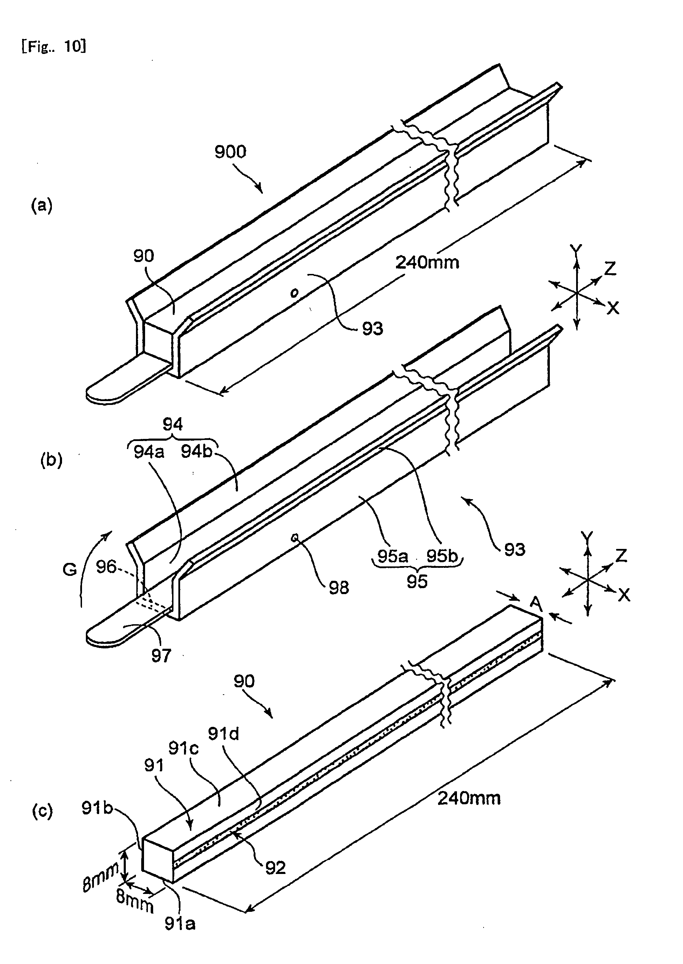

Fig. 10(a) is a perspective view of a framed excluder 900 according to a sixth embodiment of the present invention. Fig. 10(b) is a perspective view of a frame 93 of the framed excluder 900. Fig. 10(c) is a perspective view of an endoscope excluder 90 (an excluder body 91 and a contrast portion 92) in the framed excluder 900.

As shown in Fig. 10(c), the excluder body 91 in the excluder 90 has a bar shape with a square shape in transverse section (240 mm x 8 mm x 8 mm). The excluder body 91 has four side surfaces 91a, 91b, 91c, and 91d, and is compressed from the two side surfaces 91b and 91d facing each other (arrows A along the X axis).

There are provided contrast portions 92 at centers of the side surfaces 91b and 91d in the longitudinal direction (along the Z axis) of the excluder body 91. The contrast portions 92 are provided as lines of thread that are made of a roentgenopaque and thermally adhesive resin and are attached to the side surfaces 91b and 91d by thermal fusion bonding. Examples of the line of thread made of a roentgenopaque and thermally adhesive resin include a multifilament obtained by kneading barium sulfate into a polypropylene series resin, and a filum obtained by kneading barium sulfate into a vinyl chloride resin.

The contrast portion 92 may be formed on the side surfaces 91a and 91c provided in the direction perpendicular to the compression directions (along the Y axis), instead of the side surfaces 91b and 91d to be compressed.

As shown in Fig. 10(b), the frame 93 has a long body, and includes a bottom wall 96 and left and right side walls 94 and 95 linearly extending in the longitudinal direction (along the Z axis). The left and right side walls 94 and 95 are configured by support walls 94a and 95a, and wings 94b and 95b extending from upper edges of the support walls, respectively. There is formed a space surrounded by the support walls 94a and 95a and the bottom wall 96, which is as large as tightly accommodating the excluder body 91.

The frame 93 is provided at one end in the longitudinal direction with a tongue portion 97 protruding therefrom. The tongue portion 97 is provided along the bottom wall 96 so as to be raised upward from the bottom wall 96 about a pin 98 serving as a rotational axis (an arrow G).

As shown in Fig. 10(a), the framed excluder 900 includes the frame 93 and the excluder 90 that is accommodated in the inner cavity of the frame 93. The excluder 90 is supported by the support walls 94a and 95a of the frame 93 in the compression directions (the arrows A) so as not to be expanded.

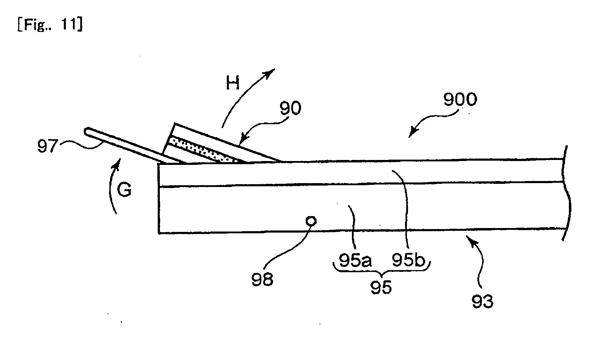

Described next is a method of using the framed excluder 900 according to the sixth embodiment. Fig. 11 is a side view showing a state where the excluder 90 is taken out of the frame 93.

Initially, the tongue portion 97 of the frame 93 is raised upward (the arrow G), and one end portion of the excluder 90 is accordingly lifted upward so as to be exposed from the frame 93. The exposed end portion is held with a hand to take the excluder 90 out of the frame 93 (an arrow H).

This excluder 90 thus taken out (see Fig. 10(c)) is inserted into a body cavity (such as a abdominal cavity or a thoracic cavity) through the cylindrical portion of the trocar. In this state, because the contrast portions 92 are attached by thermal fusion bonding to the center of the side surfaces 91b and 91d of the excluder body 91 that has a rectangular shape in section, the contrast portions 92 are not frictioned with the cylindrical inner wall of the trocar. Therefore, the contrast portions 92 are quite unlikely to be frictioned to be fallen off.

Thereafter, physiological saline is poured on the excluder 90 in the body cavity, so that the excluder 90 is swollen and increased in size to exclude an organ.

### Experiments

The excluder body of the present invention was tested to find out changes in size when processed by the gas sterilization treatment with use of ethylene oxide gas.

Adopted as the excluder bodies were cellulose sponges that were obtained by drying and compression molding at a compressibility of 10% (a test sample No. 1: 200 mm × 8 mm × 8 mm, and a test sample No. 2: 200 mm × 8 mm × 6 mm). The test sample No. 1 prior to the compression had a size of 200 mm × 8 mm × 80 mm, while the test sample No. 2 prior to the compression had a size of 200 mm × 8 mm × 60 mm. The test samples Nos. 1 and 2 were compressed respectively in the thickness direction (see the arrows A indicated in Figs. 1(a), 6(c), and 10(c)) into the sizes specified above.

Three pieces of each of the test samples Nos. 1 and 2 were inserted into a sterilization bag and were processed by the ethylene oxide gas sterilization treatment. The sizes in the thickness direction of these pieces were measured after having been processed by the sterilization treatment for four and six hours. The measurements were each performed on the most expanded portion in the thickness direction. The test results are indicated in Table 1 below.

**[Table 1]**

| Test sample | Thickness(mm) | | Time for sterilization (hour) | Thickness after sterilization (mm) [Measuring at maximum portion] | | Rate of increase (%) | |
|---|---|---|---|---|---|---|---|
| | Before | After | | | | | |
| | compression | compression | | Each sample | Average | Each sample | Average |
| No.1 | 80 | 8 | 4 | 14.0 | 14.83 | 175.00 | 185.42 |
| | | | | 15.0 | | 187.50 | |
| | | | | 15.5 | | 193.75 | |
| | | | 6 | 15.5 | 14.67 | 193.75 | 183.33 |
| | | | | 14.5 | | 181.25 | |
| | | | | 14.0 | | 175.00 | |
| No.2 | 60 | 6 | 4 | 18.0 | 18.17 | 300.00 | 302.78 |
| | | | | 18.5 | | 308.33 | |
| | | | | 18.0 | | 300.00 | |
| | | | 6 | 18.0 | 18.00 | 300.00 | 300.00 |
| | | | | 18.0 | | 300.00 | |
| | | | | 18.0 | | 300.00 | |

As apparent from Table 1, each of the pieces of the test samples was expanded by the ethylene oxide gas sterilization treatment. Such expansion results in difficulty of insertion into the cylindrical portion of the trocar. However, if the sterilization treatment is applied to the excluder body that is accommodated in the frame, such expansion can be suppressed. Consequently, it is effective to provide such a framed excluder.

The excluder as well as the framed excluder according to the present invention have been specifically described with reference to the exemplary drawings. However, the present invention should not be limited to the illustrated examples, and can be embodied with appropriate modifications as long as being applicable to the purposes of the invention. Such modifications are all included in the technical scope of the present invention.

For example, the fourth embodiment exemplified the frame 40 that is provided with the main cavity 44 identical in size with the width (6 mm) in the compression directions of the excluder body 45. Alternatively, the main cavity 44 in the frame 40 may be larger than the excluder body 45. Even in such a case, the main cavity 44 in the frame 40 is still required not to be larger than the cylindrical inner cavity 71c of the trocar. This is because the excluder body 45 needs to be sized so as to be inserted into the cylindrical inner cavity of the trocar even in a case where the excluder body 45 is swollen enough to be in contact with the inner wall surfaces of the frame 40.

Further, the fourth embodiment exemplified the rectangular recesses 42b and 43b as the grooves in the side walls 42 and 43. Alternatively, these recesses may be provided as semicircular or triangular grooves.

Moreover, in the fourth embodiment, the frame 40 may not be provided with the bottom wall 41 (but having a frame for linking the side walls 42 and 43).

The fourth embodiment exemplified the frame 40 that has the grooves (the recesses 42b and 43b) in the side walls 42 and 43. Alternatively, the frame may not be provided with the grooves in a case where adopted is an excluder having a contrast thread that does not pass through the side surfaces of an excluder body (such as an excluder having a contrast thread passing through the axis of an excluder body in a bar shape, an excluder having a contrast thread that is attached to a side surface of an excluder body by thermal fusion bonding, or an excluder having an RFID tag in place of a contrast thread attached to an excluder body in order to prevent such a contrast thread from remaining) (in the sixth embodiment, for example).

Furthermore, each of the side walls of the frame is not limited to a plate body having no pore as shown in Figs. 6(a), 6(b), and 7 to 9 as long as the side wall can prevent expansion of the excluder. For example, the side wall may be a plate having a plurality of pores or a meshed plate.

In each of the first to sixth embodiments described above, the compression directions were only aligned with the arrows A (along the X axis). Alternatively, the compression may be performed in vertical and horizontal directions (along the X axis as well as the Y axis). In this case, the frame is preferably provided with the side walls so as to correspond to these compression directions.

### DESCRIPTION OF SYMBOLS

- 10, 20, 30, 50, 90: endoscope excluder
- 15, 25, 35, 45, 91: excluder body
- 15e, 15f, 26, 27: flat portion
- 16: contrast thread
- 16a, 16b: entering/exiting portion of inserted contrast thread
- 36, 37: concave portion
- 40, 80, 93: frame
- 41, 42, 43, 94, 95: side wall
- 42a, 42c, 43a, 43c, 81a, 81c, 82a, 82c, 94a, 95a: support wall
- 42b, 43b: recess (groove)
- 44: main cavity
- 47, 48: sub cavity
- 70: trocar
- 71a: trocar cylindrical inner wall
- 92: contrast portion
- 97: tongue portion
- 98: pin
- 100, 800, 900: framed excluder

## Claims

1. An endoscope excluder (10, 20, 30, 50, 90) used in an endoscopic surgery, comprising:
an excluder body (15, 25, 35, 45, 91) made of a hygroscopically expanding material that is obtained by drying and compression molding, wherein
the excluder body (15, 25, 35, 45, 91) has a bar shape and a transverse section smaller than a section of a cylindrical inner cavity of a trocar (71), wherein
the hygroscopically expanding material is a cellulose sponge, **characterized in that**
the excluder body (15, 25, 35, 45, 91) in the bar shape is cut so as to have a longitudinal direction thereof that is aligned with a direction of extruding the cellulose sponge in a production process thereof, and
when the extrusion direction is defined to be along a Z axis and two directions perpendicular to the extrusion direction are defined to be along an X axis and a Y axis, the cellulose sponge is compressed in the directions along the X axis and/or the Y axis.

2. The endoscope excluder (10, 20, 30, 50, 90) according to claim 1, wherein
the excluder body (15, 25, 35, 45, 91) in the bar shape has a long axis that is defined to be aligned with a Z axis, and the hygroscopically expanding material is compressed in directions perpendicular to the Z axis.

3. The endoscope excluder (50, 90) according to claim 1 or 2, further comprising:
a long frame (40, 80, 93) that accommodates the excluder body (15, 45, 91) so as to be taken out thereof, wherein
sides perpendicular to the compression directions of the excluder body (15, 45, 91) are provided with paired support walls (42a, 42c, 43a, 43c, 81 a, 81 c, 82a, 82c, 94a, 95a) facing each other along the Z axis.

4. The endoscope excluder (50, 90) according to claim 3, wherein
the frame (40, 93) has an opened portion on a side other than the sides perpendicular to the compression directions.

5. The endoscope excluder (50, 90) according to claim 3, wherein
when two directions perpendicular to the Z axis are defined to be along an X axis and a Y axis,
the excluder body (15, 45, 91) is compressed along the X axis, and the frame (40, 80, 93) is opened at least on one side in the direction along the Y axis.

6. The endoscope excluder (90) according to claim 5, wherein
the frame (93) has a bottom wall (96) on another side in the direction along the Y axis, and a tongue portion (97) at least partially on an inner wall surface of the bottom wall (96) so as to be along the inner wall surface, and
the tongue portion (97) protrudes from the bottom wall (96) in one direction along the Z axis so as to be raised toward the open side in the direction along the Y axis.

7. The endoscope excluder (10, 20, 30, 50, 90) according to any one of claims 1 to 6, wherein
the excluder body (15, 25, 35, 45, 91) absorbs moisture to be expanded at an expansion factor of five times or more in the compression directions.

8. The endoscope excluder (90) according to any one of claims 1 to 7, wherein
the excluder body (91) has a roentgenopaque resin attached thereto by thermal fusion bonding.

9. The endoscope excluder (10, 20, 30, 50) according to any one of claims 1 to 7, wherein
the excluder body (15, 25, 35, 45) has a contrast thread (16) inserted there-through and knotted,
the excluder body (15, 25, 35, 45) has, on side surfaces along a longitudinal direction, two positions in total each provided with a flat portion (15e, 15f) or a concave portion (36, 37), and the contrast thread (16) is inserted through the excluder body (15) via the flat portion (15e, 15f) or the concave portion (36, 37).

10. The endoscope excluder (10, 20, 30, 50) according to claim 9, wherein
the contrast thread (16) is inserted through the cellulose sponge along the X axis or the Y axis.

11. The endoscope excluder (10, 20, 30, 50) according to claim 10, wherein
the cellulose sponge is compressed along the X axis in the process of producing the cellulose sponge, and the contrast thread (16) is inserted through the cellulose sponge along the X axis and knotted.

12. The endoscope excluder (50) according to any one of claims 9 to 11, wherein
the frame (40, 80) is provided with a groove (42b, 43b) or a gap (83, 84) along the support wall (42a, 42c, 43a, 43c, 81 a, 81 c, 82a, 82c).

## Patentansprüche

1. Endoskopausnehmer (10, 20, 30, 50, 90), verwendet in einer endoskopischen Operation, umfassend:
einen Ausnehmerkörper (15, 25, 35, 45, 91), hergestellt aus einem sich hygroskopisch ausdehnenden Material, das erhalten ist durch Trocknen und Formpressen, wobei
der Ausnehmerkörper (15, 25, 35, 45, 91) eine Stabform und einen Querschnitt aufweist, der kleiner als ein Bereich eines zylindrischen inneren Hohlraums eines Trokars (71) ist, wobei
das sich hygroskopisch ausdehnende Material ein Zelluloseschwamm ist, **dadurch gekennzeichnet, dass**
der Ausnehmerkörper (15, 25, 35, 45, 91) in der Stabform derart geschnitten ist, um eine Längsrichtung davon aufzuweisen, die mit einer Richtung des Extrudierens des Zelluloseschwammes in einem Herstellungsverfahren davon ausgerichtet ist, und
wenn die Extrusionsrichtung definiert ist, entlang einer Z-Achse zu sein, und zwei Richtungen senkrecht zu der Extrusionsrichtung definiert sind, entlang einer X-Achse und einer Y-Achse zu sein, der Zelluloseschwamm in den Richtungen entlang der X-Achse und/oder der Y-Achse zusammengepresst ist.

2. Endoskopausnehmer (10, 20, 30, 50, 90) nach Anspruch 1, wobei
der Ausnehmerkörper (15, 25, 35, 45, 91) in der Stabform eine lange Achse aufweist, die definiert ist, mit einer Z-Achse ausgerichtet zu sein, und das sich hygroskopisch ausdehnende Material in Richtungen senkrecht zu der Z-Achse zusammengepresst ist.

3. Endoskopausnehmer (50, 90) nach Anspruch 1 oder 2, weiter umfassend:
einen Langrahmen (40, 80, 93), der den Ausnehmerkörper (15, 45, 91) derart enthält, um daraus entnommen zu werden, wobei
Seiten senkrecht zu den Pressrichtungen des Ausnehmerkörpers (15, 45, 91) mit paarweisen Tragwänden (42a, 42c, 43a, 43c, 81 a, 81c, 82a, 82c, 94a, 95a) bereitgestellt sind, die sich entlang der Z-Achse gegenüberliegen.

4. Endoskopausnehmer (50, 90) nach Anspruch 3, wobei
der Rahmen (40, 93) auf einer von zu den Pressrichtungen senkrechten Seiten verschiedenen Seite einen offenen Bereich aufweist.

5. Endoskopausnehmer (50, 90) nach Anspruch 3, wobei
wenn zwei Richtungen senkrecht zu der Z-Achse definiert sind, entlang einer X-Achse und einer Y-Achse zu sein,
der Ausnehmerkörper (15, 45, 91) entlang der X-Achse zusammengepresst ist, und der Rahmen (40, 80, 93) mindestens auf einer Seite in der Richtung entlang der Y-Achse offen ist.

6. Endoskopausnehmer (90) nach Anspruch 5, wobei
der Rahmen (93) eine Bodenwand (96) auf einer anderen Seite in der Richtung entlang der Y-Achse und einen Zungenteil (97) mindestens teilweise auf einer Innenwandoberfläche der Bodenwand (96) derart aufweist, um entlang der Innenwandoberfläche zu sein, und
der Zungenteil (97) von der Bodenwand (96) in eine Richtung entlang der Z-Achse derart vorsteht, um in Richtung der offenen Seite in der Richtung entlang der Y-Achse angehoben zu werden.

7. Endoskopausnehmer (10, 20, 30, 50, 90) nach einem der Ansprüche 1 bis 6, wobei
der Ausnehmerkörper (15, 25, 35, 45, 91) Feuchtigkeit absorbiert, um mit einem Ausdehnungsfaktor von Fünfmal oder mehr in den Pressrichtungen ausgedehnt zu werden.

8. Endoskopausnehmer (90) nach einem der Ansprüche 1 bis 7, wobei der Ausnehmerkörper (91) ein röntgenopakes Harz aufweist, das daran durch Wärmeschmelzverbinden angebracht ist.

9. Endoskopausnehmer (10, 20, 30, 50) nach einem der Ansprüche 1 bis 7, wobei
der Ausnehmerkörper (15, 25, 35, 45) einen Kontrastfaden (16) aufweist, der durch diesen geführt und verknotet ist,
der Ausnehmerkörper (15, 25, 35, 45) auf Seitenoberflächen entlang einer Längsrichtung insgesamt zwei Bereiche aufweist, die jeweils mit einem flachen Bereich (15e, 15f) oder einem konkaven Bereich (36, 37) bereitgestellt sind, und der Kontrastfaden (16) über den flachen Bereich (15e, 15f) oder den konkaven Bereich (36, 37) durch den Ausnehmerkörper (15) geführt ist.

10. Endoskopausnehmer (10, 20, 30, 50) nach Anspruch 9, wobei
der Kontrastfaden (16) entlang der X-Achse oder der Y-Achse durch den Zelluloseschwamm geführt ist.

11. Endoskopausnehmer (10, 20, 30, 50) nach Anspruch 10, wobei
der Zelluloseschwamm in dem Verfahren des Herstellens des Zelluloseschwammes entlang der X-Achse zusammengepresst ist, und der Kontrastfaden (16) entlang der X-Achse durch den Zelluseschwamm geführt und verknotet ist.

12. Endoskopausnehmer (50) nach einem der Ansprüche 9 bis 11, wobei
der Rahmen (40, 80) mit einer Vertiefung (42b, 43b) oder einer Spalte (83, 84) entlang der Tragwand (42a, 42c, 43a, 43c, 81 a, 81 c, 82a, 82c) bereitgestellt ist.

## Revendications

1. Ecarteur pour endoscope (10, 20, 30, 50, 90) utilisé en chirurgie endoscopique, comprenant :
un corps écarteur (15, 25, 35, 45, 91) constitué d'un matériau expansible par hygroscopie, qui est obtenu par séchage et moulage par compression, où le corps écarteur (15, 25, 35, 45, 91) à la forme d'un barreau et une coupe transversale plus petite que la coupe d'une cavité cylindrique intérieure d'un trocart (71), où le matériau expansible par hygroscopie est une éponge de cellulose, **caractérisé en ce que**
le corps écarteur (15, 25, 35, 45, 91) en forme de barreau est coupé de sorte que sa direction longitudinale soit alignée avec la direction d'extrusion de l'éponge de cellulose au cours de son procédé de production, et
lorsque la direction d'extrusion est définie comme étant l'axe Z et les deux directions perpendiculaires à la direction d'extrusion sont définies comme étant l'axe X et l'axe Y, l'éponge de cellulose est compressée dans les directions de l'axe X et/ou de l'axe Y.

2. Ecarteur pour endoscope (10, 20, 30, 50, 90) selon la revendication 1, où le corps écarteur (15, 25, 35, 45, 91) en forme de barreau a un axe long qui est défini comme étant aligné avec l'axe Z, et le matériau hygroscopique expansible est compressé dans les directions perpendiculaires à l'axe Z.

3. Ecarteur pour endoscope (50, 90) selon la revendication 1 ou 2, comprenant en outre :
un cadre long (40, 80, 93) qui accueille le corps écarteur (15, 45, 91) de manière à en être retiré, où les côtés perpendiculaires aux directions de compression du corps de banc extracteur (15, 45, 91) sont munis de parois de support par paires (42a, 42c, 43a, 43c, 81 a, 81 c, 82a, 82c, 94a, 95c) se faisant face selon l'axe Z.

4. Ecarteur pour endoscope (50, 90) selon la revendication 3, où le cadre (40, 93) présente une partie ouverte sur un côté autre que les côtés perpendiculaires aux directions de compression.

5. Ecarteur pour endoscope (50, 90) selon la revendication 3, où lorsque les deux directions perpendiculaires à l'axe Z sont définie comme l'axe X et l'axe Y, le corps écarteur (15, 45, 91) est compressé selon l'axe X et le cadre (40, 80, 93) est ouvert au moins sur un côté dans la direction de l'axe Y.

6. Ecarteur pour endoscope (90) selon la revendication 5, où le cadre présente une paroi de fond (96) sur un autre côté dans la direction de l'axe Y, et une partie de languette (97) au moins partiellement sur la surface intérieure de la paroi de fond (96) de manière à se trouver le long de la surface intérieure de la paroi, et
la partie de languette (97) fait saillie depuis la paroi de fond (96) dans la direction de l'axe Z de manière à être levée vers le côté ouvert dans la direction de l'axe Y.

7. Ecarteur pour endoscope (10, 20, 30, 50, 90) selon l'une quelconque des revendications 1 à 6, où le corps écarteur (15, 25, 35, 45, 91) absorbe l'humidité pour s'étendre d'un facteur d'expansion de cinq fois ou plus dans les directions de compression.

8. Ecarteur pour endoscope (90) selon l'une quelconque des revendications 1 à 7, où le corps écarteur (91) présente une résine opaque aux rayons X attachée à celui-ci par fusion thermique.

9. Ecarteur pour endoscope (10, 20, 30, 50) selon l'une quelconque des revendications 1 à 7, où le corps écarteur (15, 25, 35, 45) présente un filament de contraste (16) inséré dans celui-ci et noué,
le corps écarteur (15, 25, 35, 45) présente sur les surfaces latérales dans la direction longitudinale, deux positions au total, munies chacune d'une partie plane (15e, 15f) ou d'une partie concave (36, 37), et le filament de contraste (16) est inséré à travers le corps écarteur (15) via la partie plane (15e, 15f) ou la partie concave (36, 37).

10. Ecarteur pour endoscope (10, 20, 30, 50) selon la revendication 9, où le filament de contraste (16) est inséré à travers l'éponge de cellulose selon l'axe X ou l'axe Y.

11. Ecarteur pour endoscope (10, 20, 30, 50) selon la revendication 10, où l'éponge de cellulose est compressée selon l'axe X dans le procédé de production de l'éponge de cellulose, et le filament de contraste (16) est inséré à travers l'éponge de cellulose selon l'axe X et noué.

12. Ecarteur pour endoscope (50) selon l'une quelconque des revendications 9 à 11, où le cadre (40, 80) est muni d'une glissière (42b, 43b) ou d'une rainure (83, 84) le long de la paroi de support (42a, 42c, 43a, 43c, 81a, 81c, 82a, 82c).
